# EUROPEAN PATENT APPLICATION

(11) **EP 4 703 752 A1**
(43) Date of publication of application: **04.03.2026**
(21) Application number: 25194175.3
(22) Date of filing: 05.08.2025
(51) Int. Cl.: G01R 33/36

(54) **SYSTEM AND METHOD FOR CONNECTION COMPATABILITY FOR MEDICAL IMAGING**

(30) Priority: 30.08.2024 US 202418821853
(71) Applicant: GE Precision Healthcare LLC, Waukesha, WI 53188 (US)
(72) Inventor: RODEHED, Mattias, 182 32 Danderyd (SE); GUALTIERI, James William, Glenshaw, 15224 (US); VIERCK, Doug, Waukesha, 53188 (US); STEINGRABER, Ethan, Waukesha, 53188 (US)
(74) Representative: Kilburn & Strode LLP

(57) **Abstract**

Systems and methods are provided for a graphical user interface (GUI) for a magnetic resonance imaging (MRI) system. In an example, a system includes a display device (420); one or more processors (404); and memory (406) storing instructions executable by the one or more processors to: determine (906) a connection status for each of a plurality of ports (27), each port of the plurality of ports configured to couple a radiofrequency (RF) coil (14) to an MRI apparatus (10); generate a GUI (501) that includes a port status indicator (520, 522, 524, 526) for each port, each port status indicator having a visual appearance that is based on that port's connection status; and output (906) the GUI for display on the display device.

## Description

### TECHNICAL FIELD

The present description relates generally to medical imaging. More specifically, the present disclosure relates to visually presenting coil-port connectivity status in magnetic resonance imaging.

### BACKGROUND

Magnetic resonance imaging (MRI) is a medical imaging modality that can create images of the inside of a human body without using x-rays or other ionizing radiation. An MRI scan typically includes a series of radiofrequency (RF) excitation pulses and magnetic field gradient pulses that are played out with specific timings and in a specific sequence to prepare contrast and encode spatial information into an MR signal that is detected by one or more RF coils to generate an image.

In some examples, the RF coils used to receive the MR signals may be local or surface RF coil arrays that may be placed on or over an imaging subject. Such coil arrays may be adjustable in size, position, and/or orientation. For example, based a given imaging objective, an operator may position a selected surface RF coil over an imaging subject and plug the RF coil into the MRI system.

### BRIEF DESCRIPTION

In one example, a system includes a display device; one or more processors; and memory storing instructions executable by the one or more processors to: determine a connection status for each of a plurality of ports, each port of the plurality of ports configured to couple a radiofrequency (RF) coil to a magnetic resonance imaging (MRI) apparatus; generate a graphical user interface (GUI) that includes a port status indicator for each port, each port status indicator having a visual appearance that is based on that port's connection status; and output the GUI for display on the display device.

In another example, a method includes determining a connection status for each of a plurality of ports of a magnetic resonance imaging (MRI) apparatus, each port of the plurality of ports configured to couple a radiofrequency (RF) coil to the MRI apparatus, generating a graphical user interface (GUI) that includes a port status indicator for each port, each port status indicator having a visual appearance that is based on that port's connection status, and outputting the GUI for display on a display device.

In a further example, a method includes determining, based on signals output by a first port of a plurality of ports positioned on a table of a magnetic resonance imaging (MRI) apparatus, that a first RF coil is connected to the first port; determining, based on the signals, that the first RF coil is not compatible with the first port; updating, in response to determining that the first RF coil is not compatible with the first port, a graphical user interface (GUI) to include a first port status indicator for the first port having a second visual appearance that is different than a first visual appearance of the first port status indicator, the first port status indicator displayed on the GUI with the first visual appearance when the first port is determined to be idle; and outputting the GUI for display on a display device positioned on a bore of the MRI apparatus.

It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present disclosure will be better understood from reading the following description of non-limiting embodiments, with reference to the attached drawings, wherein below:
FIG. 1 is a block diagram of an example MRI apparatus;
FIG. 2 schematically shows an overhead view of an MRI system including the MRI apparatus of FIG. 1;
FIG. 3 schematically shows a front view of the MRI system of FIG. 2;
FIG. 4 schematically shows an example scan control device of the MRI apparatus of FIG. 1;
FIG. 5 shows a first view of an example in-room display (IRD) graphical user interface (GUI) that may be displayed on a display device of the MRI system of FIGS. 2 and 3;
FIG. 6 shows a second view of the example IRD GUI;
FIG. 7 shows a third view of the example IRD GUI;
FIG. 8 shows a fourth view of the example IRD GUI;
FIG. 9 is a high level flow chart illustrating a method for scanning a patient using the MRI system of FIGS. 2 and 3;
FIG. 10 is a flow chart illustrating a method for monitoring coil-port connections during the method of FIG. 9; and
FIG. 11 shows a fifth view of the example IRD GUI.

### DETAILED DESCRIPTION

The following description relates to automatic monitoring and notification of compatible and incompatible radiofrequency (RF) coil and port connections in magnetic resonance imaging (MRI) systems. During a scan of an imaging subject, such as a patient, with an MRI system, one or more RF coils may be arranged around the patient's body. Each RF coil may include a plurality of coil elements. Each coil element is configured to send MR signals to the MRI system, via a channel of a plurality of channels, for eventual processing into an image. Each RF coil may have a cable that is connected to each of the coil elements of the RF coil and that terminates at a connector. The connector is configured to couple with a corresponding port of the MRI system, so that the MR signals may be transmitted from the coil elements to the MRI system. Different anatomical features that are to be imaged may dictate different coil element configurations, and thus various different RF coils are available to be used during a scan. For example, some RF coils may include more coil elements than other RF coils. The different RF coils may be configured to send the MR signals over different numbers of channels. For example, some RF coils may be configured with 16 channels while other RF coils may be configured with 21 channels. Further, some RF coils may be configured as receive-only RF coils (e.g., that are only configured to receive and send the MR signals to the MRI system) while other RF coils may be configured as transmit/receive RF coils (e.g., that are configured to both transmit RF signals and receive and send the detected MR signals to the MRI system). Thus, MRI systems may likewise include multiple different ports configured to receive the different types of RF coils.

However, typically, the ports of the MRI system may appear identical to each other. Likewise, the connectors of the RF coils may appear identical to each other. Thus, even when a give port is configured to couple to a particular type of RF coil (e.g., having 16 channels), the physical configurations of the port and RF coils may allow the port to be coupled to various different types of RF coils. As such, during patient set-up prior to scanning, an operator of the MRI system may accidentally plug an RF coil into a port that is not compatible with the RF coil (e.g., the operator may plug a 21 channel RF coil into a port configured to accept 16 channel RF coils). The operator may not be aware of the error until the operator attempts to initiate scanning and images cannot be obtained, or poor quality images are obtained. Due to the distribution of the components of the MRI system across the scanning environment, once an RF coil-port misconnection is identified, the operator may have to stop scanning, move from a remote scan control room to the scan room actually housing the scanner of the MRI system, and attempt to plug the RF coil into the right port. Even once a misconnection is identified, fixing the error may be time-consuming, given that the operator may not be aware of which port(s) is actually configured to accept the RF coil. As such, incompatible connections between RF coils and ports of the MRI system may delay scanning and/or result in unnecessary scanning of a patient.

Thus, according to embodiments disclosed herein, the MRI system may automatically monitor each port of the MRI system to identify when a connection between a port and an RF coil has been established. The MRI system may be configured to determine if a given RF coil-port connection is compatible or incompatible for scanning. The MRI system may display a graphical user interface (GUI) on one or more display devices that includes visual depictions of the ports of the MRI system. When an RF coil-port connection is identified, the visual depiction of that port may be updated to indicate if the connection is compatible or incompatible. Further, when an incompatible connection is detected, additional information about the incompatible connection may be displayed on the GUI to inform the operator of the reason for the incompatible connection and direct the operator to a port that is compatible with the RF coil.

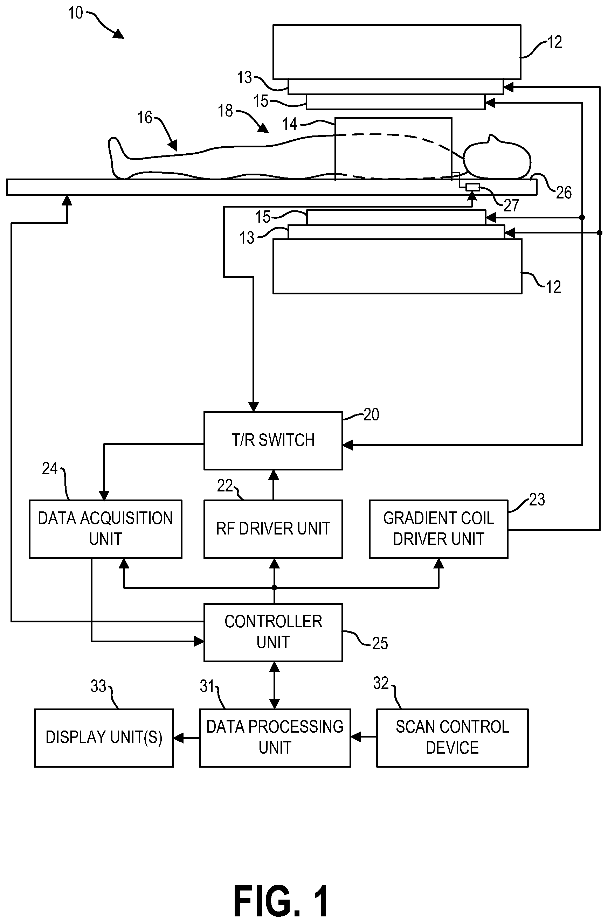

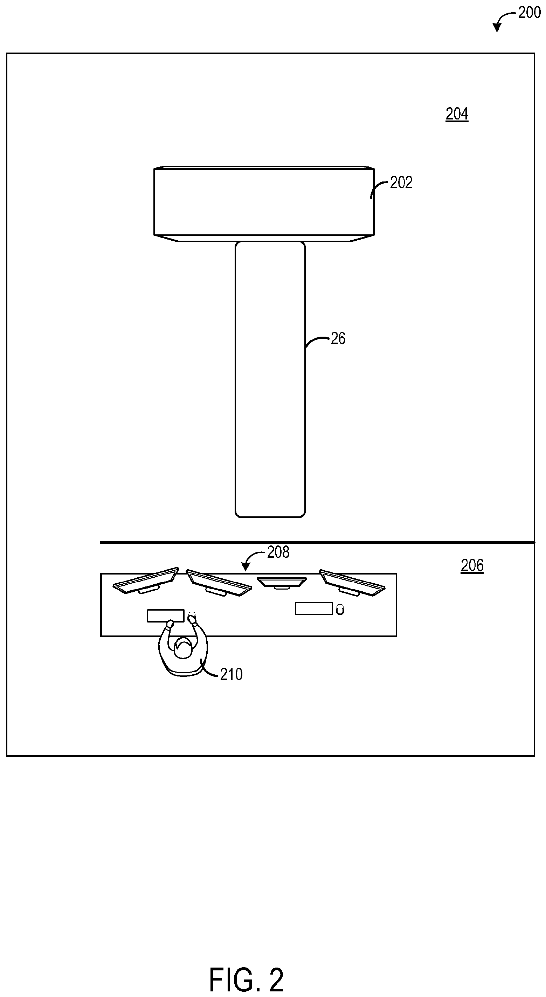

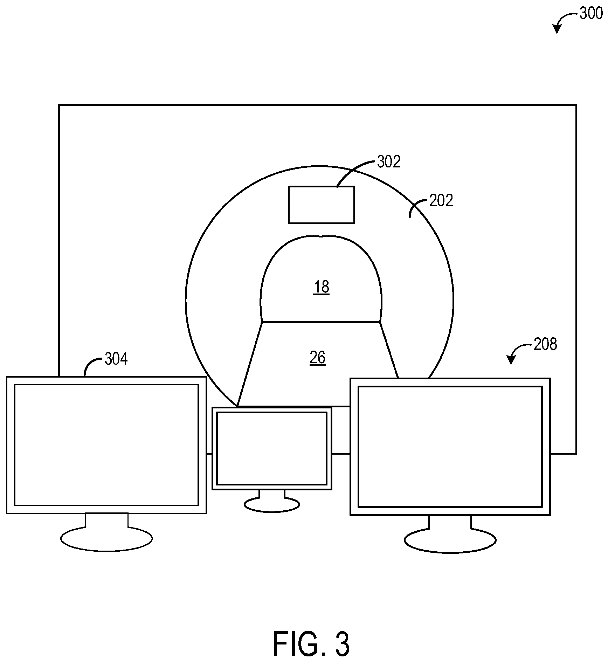

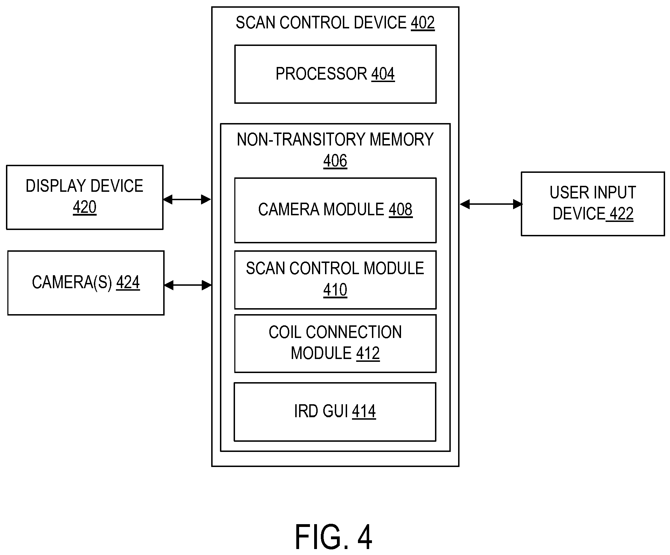

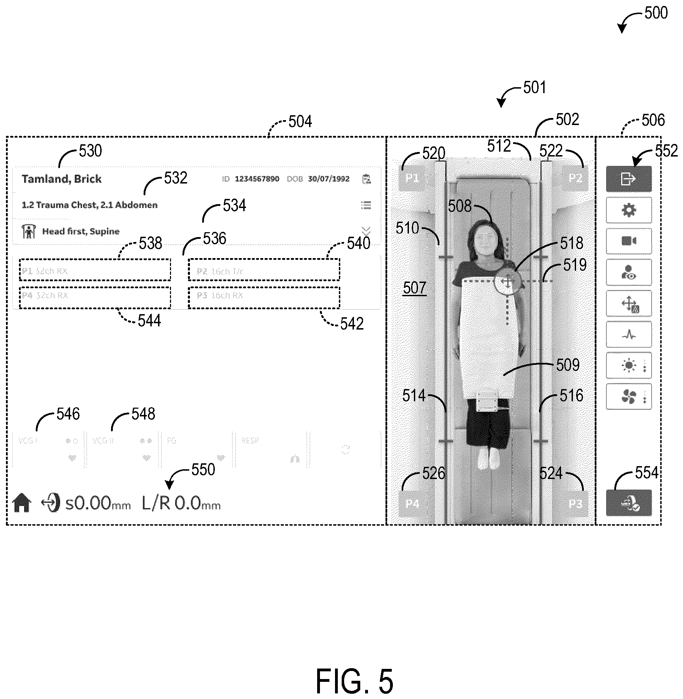

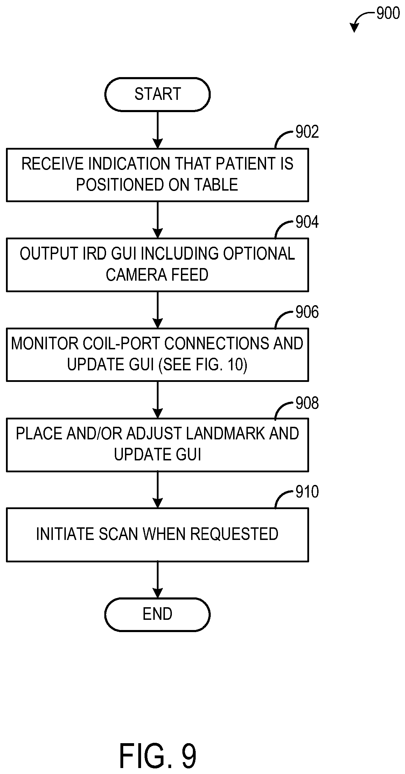

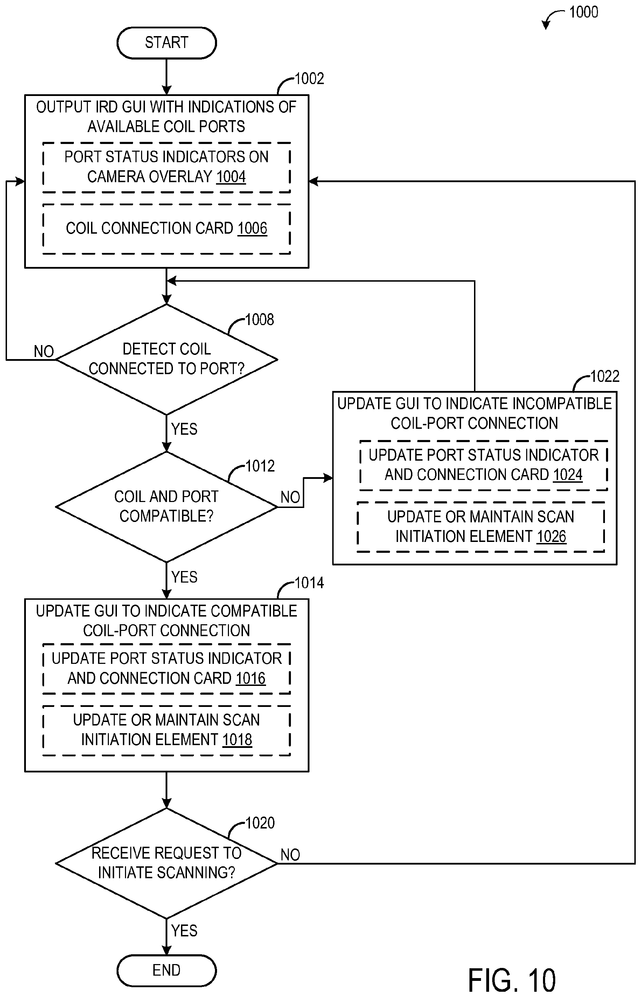

An example MRI apparatus that may be used to obtain MR signals of an imaging subject using one or more RF coils is shown in FIG. 1. The MRI apparatus may be included as part of an MRI system that includes various display devices distributed across a scanning environment, as shown in FIGS. 2 and 3. The MRI apparatus may include a scan control device, such as the scan control device of FIG. 4, configured to control at least some scan parameters of the MRI apparatus (e.g., command the MRI apparatus to perform a scan of a patient according to a selected scan protocol) and monitor connections between RF coils and connection ports of the MRI apparatus, according to the methods of FIGS. 9 and 10. The scan control device may be configured to generate a GUI that may be displayed on one or more display devices of the MRI system. The GUI may be updated based on current RF coil-port connections in order to notify a user of compatible and incompatible RF coil-port connections, as shown in FIGS. 5-8 and 11.

FIG. 1 illustrates an MRI apparatus 10 (e.g., an MRI system) that includes a magnetostatic field magnet unit 12, a gradient coil unit 13, an RF coil unit 14, an RF body coil unit 15 (e.g., volume coil unit), a transmit/receive (T/R) switch 20, an RF driver unit 22, a gradient coil driver unit 23, a data acquisition unit 24, a controller unit 25, a patient bed or table 26, a data processing unit 31, a scan control device 32, and one or more display units 33. In some embodiments, the RF coil unit 14 is a surface coil, which is a local coil typically placed proximate to the anatomy of interest of a subject 16. Herein, the RF body coil unit 15 is a transmit coil that transmits RF signals, and the local surface of the RF coil unit 14 receives the MR signals. As such, the transmit body coil (e.g., RF body coil unit 15) and the surface receive coil (e.g., RF coil unit 14) are separate but electromagnetically coupled components. The MRI apparatus 10 transmits electromagnetic pulse signals to the subject 16 placed in an imaging space 18 with a static magnetic field formed to perform a scan for obtaining magnetic resonance signals from the subject 16. One or more images of the subject 16 can be reconstructed based on the magnetic resonance signals thus obtained by the scan.

The magnetostatic field magnet unit 12 includes, for example, an annular superconducting magnet, which is mounted within a toroidal vacuum vessel. The magnet defines a cylindrical space surrounding the subject 16 and generates a constant primary magnetostatic field ***B₀.***

The MRI apparatus 10 also includes a gradient coil unit 13 that forms a gradient magnetic field in the imaging space 18 so as to provide the magnetic resonance signals received by the RF coil arrays with three-dimensional positional information. The gradient coil unit 13 includes three gradient coil systems, each of which generates a gradient magnetic field along one of three spatial axes perpendicular to each other, and generates a gradient field in each of a frequency encoding direction, a phase encoding direction, and a slice selection direction in accordance with the imaging condition. More specifically, the gradient coil unit 13 applies a gradient field in the slice selection direction (or scan direction) of the subject 16, to select the slice; and the RF body coil unit 15 or the local RF coil arrays may transmit an RF pulse to a selected slice of the subject 16. The gradient coil unit 13 also applies a gradient field in the phase encoding direction of the subject 16 to phase encode the magnetic resonance signals from the slice excited by the RF pulse. The gradient coil unit 13 then applies a gradient field in the frequency encoding direction of the subject 16 to frequency encode the magnetic resonance signals from the slice excited by the RF pulse.

The RF coil unit 14 is disposed, for example, to enclose the region to be imaged of the subject 16. In some examples, the RF coil unit 14 may be referred to as the surface coil or the receive coil. In the static magnetic field space or imaging space 18 where a static magnetic field ***B₀*** is formed by the magnetostatic field magnet unit 12, the RF body coil unit 15 transmits, based on a control signal from the controller unit 25, an RF pulse that is an electromagnet wave to the subject 16 and thereby generates a high-frequency magnetic field ***B₁.*** This excites a spin of protons in the slice to be imaged of the subject 16. The RF coil unit 14 receives, as a magnetic resonance signal, the electromagnetic wave generated when the proton spin thus excited in the slice to be imaged of the subject 16 returns into alignment with the initial magnetization vector. In some embodiments, the RF coil unit 14 may transmit the RF pulse and receive the MR signal. In other embodiments, the RF coil unit 14 may only be used for receiving the MR signals, but not transmitting the RF pulse. The RF coil unit 14 may be coupled to the MRI apparatus 10 via a port 27. For example, the RF coil unit 14 may include a cable with a connector configured to be positioned within the port 27 so that the MR signals obtained with the RF coil unit 14 may be transmitted to the data acquisition unit 24 (explained in more detail below). It is to be appreciated that while FIG. 1 shows one RF coil unit 14 and one port 27, the table 26 may include multiple ports (e.g., two ports, four ports) each configured to accept a connector of a respective RF coil unit. Different types of RF coil units may be utilized during a scan depending on the anatomy being imaged, size of the subject, etc. RF coil units may vary by number and placement of coil elements as well as the number of channels and whether the RF coil unit is configured to only receive MR signals, or to also transmit RF signals. Likewise, the ports available for connecting to RF coil units may vary by the number of channels the ports are configured to accept/connect with and whether the port is configured to only receive signals from an RF coil unit or whether the port can also send signals to command transmission of RF signals. For example, a first port may be configured to connect to RF coil units that have 32 or fewer channels (in receive only mode), a second port may be configured to connect to RF coil units that have 16 or fewer channels (in receive only mode), and a third port may be configured to connect to RF coil units that have 16 or fewer channels and that can operate in receive mode and in transmit mode.

The RF body coil unit 15 is disposed, for example, to enclose the imaging space 18, and produces RF magnetic field pulses orthogonal to the main magnetic field ***B₀*** produced by the magnetostatic field magnet unit 12 within the imaging space 18 to excite the nuclei. In contrast to the RF coil unit 14, which may be disconnected from the MRI apparatus 10 and replaced with another RF coil unit, the RF body coil unit 15 is fixedly attached and connected to the MRI apparatus 10. Furthermore, whereas local coils such as the RF coil unit 14 can transmit to or receive signals from only a localized region of the subject 16, the RF body coil unit 15 generally has a larger coverage area. The RF body coil unit 15 may be used to transmit or receive signals to the whole body of the subject 16, for example. Using receive-only local coils and transmit body coils provides a uniform RF excitation and good image uniformity at the expense of high RF power deposited in the subject. For a transmit-receive local coil, the local coil provides the RF excitation to the region of interest and receives the MR signal, thereby decreasing the RF power deposited in the subject. It should be appreciated that the particular use of the RF coil unit 14 and/or the RF body coil unit 15 depends on the imaging application.

The T/R switch 20 can selectively electrically connect the RF body coil unit 15 to the data acquisition unit 24 when operating in receive mode, and to the RF driver unit 22 when operating in transmit mode. Similarly, the T/R switch 20 can selectively electrically connect the RF coil unit 14 to the data acquisition unit 24 when the RF coil unit 14 operates in receive mode, and to the RF driver unit 22 when operating in transmit mode. When the RF coil unit 14 and the RF body coil unit 15 are both used in a single scan, for example if the RF coil unit 14 is configured to receive MR signals and the RF body coil unit 15 is configured to transmit RF signals, then the T/R switch 20 may direct control signals from the RF driver unit 22 to the RF body coil unit 15 while directing received MR signals from the RF coil unit 14 to the data acquisition unit 24. The coils of the RF body coil unit 15 may be configured to operate in a transmit-only mode or a transmit-receive mode. The coils of the RF coil unit 14 may be configured to operate in a transmit-receive mode or a receive-only mode.

The RF driver unit 22 includes a gate modulator (not shown), an RF power amplifier (not shown), and an RF oscillator (not shown) that are used to drive the RF coils (e.g., RF body coil unit 15) and form a high-frequency magnetic field in the imaging space 18. The RF driver unit 22 modulates, based on a control signal from the controller unit 25 and using the gate modulator, the RF signal received from the RF oscillator into a signal of predetermined timing having a predetermined envelope. The RF signal modulated by the gate modulator is amplified by the RF power amplifier and then output to the RF body coil unit 15.

The gradient coil driver unit 23 drives the gradient coil unit 13 based on a control signal from the controller unit 25 and thereby generates a gradient magnetic field in the imaging space 18. The gradient coil driver unit 23 includes three systems of driver circuits (not shown) corresponding to the three gradient coil systems included in the gradient coil unit 13.

The data acquisition unit 24 includes a pre-amplifier (not shown), a phase detector (not shown), and an analog/digital converter (not shown) used to acquire the magnetic resonance signals received by the RF coil unit 14. In the data acquisition unit 24, the phase detector phase detects, using the output from the RF oscillator of the RF driver unit 22 as a reference signal, the magnetic resonance signals received from the RF coil unit 14 and amplified by the pre-amplifier, and outputs the phase-detected analog magnetic resonance signals to the analog/digital converter for conversion into digital signals. The digital signals thus obtained are output to the data processing unit 31.

The MRI apparatus 10 includes a table 26 for placing the subject 16 thereon. The subject 16 may be moved inside and outside the imaging space 18 by moving the table 26 based on control signals from the controller unit 25.

The controller unit 25 includes a computer and a recording medium on which a program to be executed by the computer is recorded. The program when executed by the computer causes various parts of the apparatus to carry out operations corresponding to pre-determined scanning. The recording medium may comprise, for example, a ROM, flexible disk, hard disk, optical disk, magneto-optical disk, CD-ROM, or non-volatile memory card. The controller unit 25 is connected to the scan control device 32 and processes the operation signals input to the scan control device 32 and furthermore controls the table 26, RF driver unit 22, gradient coil driver unit 23, and data acquisition unit 24 by outputting control signals to them. The controller unit 25 also controls, to obtain a desired image, the data processing unit 31 and the one or more display units 33 based on operation signals received from the scan control device 32.

The scan control device 32 includes user input devices such as a touchscreen, keyboard and a mouse. The scan control device 32 is used by an operator, for example, to input such data as an imaging protocol and to set a region where an imaging sequence is to be executed. The data about the imaging protocol and the imaging sequence execution region are output to the controller unit 25.

The data processing unit 31 includes a computer and a recording medium on which a program to be executed by the computer to perform predetermined data processing is recorded. The data processing unit 31 is connected to the controller unit 25 and performs data processing based on control signals received from the controller unit 25. The data processing unit 31 is also connected to the data acquisition unit 24 and generates spectrum data by applying various image processing operations to the magnetic resonance signals output from the data acquisition unit 24.

The one or more display units 33 include at least one display device that displays an image on a display screen of the display device based on control signals received from the controller unit 25. The one or more display units 33 may display, for example, an image regarding an input item about which the operator inputs operation data from the scan control device 32. The one or more display units 33 may also display a two-dimensional (2D) slice image or three-dimensional (3D) image of the subject 16 generated by the data processing unit 31.

During an MRI scan using the MRI apparatus 10, a subject may be positioned within the imaging space 18 and an acquisition protocol may be carried out to obtain MR signals of the subject. The acquisition protocol may include a plurality of pulse sequences where in each pulse sequence, contrast is prepared via one or more RF pulses applied by the RF body coil unit 15 and the gradient coil unit 13 is controlled to spatially encode the resultant MR signals. The spatially-encoded MR signals are received by the RF coil unit 14 are digitized and stored in k-space. Thus, k-space data or a k-space dataset may refer to the raw MR signals prior to processing into an image. In some examples, one line of k-space may be filled with the raw MR signals per pulse sequence (also referred to as repetition time). In other examples, one line of k-space may be filled with the raw MR signals per echo, where more than one echo is generated per pulse sequence/repetition time. The k-space data may also be referred to as imaging data or MR data herein.

As described above, the MRI apparatus 10 may include one or more display units 33. In examples disclosed herein, the one or more display units 33 may include an in-room display (IRD) that is positioned on a bore that houses certain elements of the MRI apparatus 10 (e.g., the magnetostatic field magnet unit 12, the gradient coil unit 13, and the RF body coil unit 15). Due to the strong magnetic fields generated during an MRI scan, an operator of the MRI apparatus 10 may be positioned remotely from the bore during active scanning. As such, the one or more display devices may further include an operator console positioned in an operator control room.

FIG. 2 schematically shows a top view 200 of the MRI apparatus 10 including a bore 202 and the table 26 positioned in a scan room 204. Adjacent the scan room 204 is an operator control room 206. The operator control room 206 may include a plurality of display devices 208. The operator control room 206 may be separated from the scan room 204 by a wall, which may include a window to allow an operator 210 to view the scan room 204, and specifically the bore 202, table 26, and the imaging subject positioned thereon. FIG. 3 schematically shows a front view 300 of the MRI apparatus 10, depicting a view the operator 210 may have when positioned in the operator control room 206. The bore 202 may house the magnetostatic field magnet unit 12, the gradient coil unit 13, and the RF body coil unit 15 and various electrical connections. In some examples, the T/R switch 20, RF driver unit 22, gradient coil driver unit 23, data acquisition unit 24, and/or controller unit 25 may also be housed in the bore 202. The bore 202 includes an opening that forms the imaging space 18 and the table 26 is configured to move in and out of the opening/imaging space 18 to scan the subject.

Prior to initiating an MRI scan, the operator 210 may be positioned in the scan room 204 with the subject (not shown in FIG. 2), in order to assist with positioning of the subject on the table 26 and place one or more RF coil units (such as RF coil unit 14) on the subject. Once positioned, the operator 210 may connect each RF coil unit to a respective port on the table 26, such as port 27. When scanning is initiated, the table 26 may move into the bore 202 and the operator 210 may move to the operator control room 206.

Various aspects of the scan may be visualized and controlled via the one or more display units 33. For example, as shown in FIG. 3, an IRD 302 is positioned on the bore 202. Further, the plurality of display devices 208 in the operator control room 206 may include an operator console 304. Each of the IRD 302 and the operator console 304 are non-limiting examples of the one or more display units 33, and each may be communicatively connected to the scan control device 32. As explained in more detail below, the IRD 302 may display an IRD graphical user interface (GUI) that visualizes a limited amount of information relevant to initiating a scan, such as information related to positioning the subject, coil placement, landmarking, and the like. The IRD GUI may include user interface elements (e.g., buttons) configured to receive user input (e.g., touch input) in order to place landmarks, initiate scanning, and so forth. The operator console 304 may display a scan control GUI that presents information and accepts user input related to the scan, in order to allow selection of a scan protocol, view localizer scan images, set scan parameters, view scan progress, view diagnostic images, and the like. As non-limiting examples, the plurality of display devices 208 of the operator control room 206 may additionally include a display device communicatively coupled to a picture archiving and communication system (PACS), radiology information system (RIS), and/or electronic medical record (EMR) system, and a display device communicatively coupled to a contrast monitoring device.

Referring to FIG. 4, scan control device 402 configured to control scan parameters of an MRI scan is shown. In some embodiments, scan control device 402 is incorporated into the MRI apparatus 10. For example, scan control device 402 may be provided in the MRI apparatus 10 as scan control device 32. In some embodiments, at least a portion of scan control device 402 is disposed at a device (e.g., edge device, server, etc.) communicably coupled to the MRI apparatus 10 via wired and/or wireless connections. In some embodiments, at least a portion of scan control device 402 is disposed at a separate device (e.g., a workstation) which can communicate with the controller unit of the MRI apparatus and/or the IRD 302, for example. Scan control device 402 may be operably/communicatively coupled to a user input device 422, at least one display device 420, and one or more cameras 424. In some examples, the user input device 422 may be the user input device of scan control device 32, explained above. Likewise, display device 420 may be the one or more display units 33 of MRI apparatus 10, such as the IRD 302. The one or more cameras 424 may include one or more visible light cameras (e.g., RGB or monochrome cameras) and/or one or more depth cameras positioned in the scan room (e.g., in scan room 204). The one or more cameras 424 may be positioned to image the table and subject (e.g., table 26 and subject 16) prior to and/or during a scan with the MRI apparatus.

Scan control device 402 includes one or more processors, such as processor 404, configured to execute machine readable instructions stored in non-transitory memory 406. Processor 404 may be single core or multi-core, and the programs executed thereon may be configured for parallel or distributed processing. In some embodiments, processor 404 may optionally include individual components that are distributed throughout two or more devices, which may be remotely located and/or configured for coordinated processing. In some embodiments, one or more aspects of processor 404 may be virtualized and executed by remotely-accessible networked computing devices configured in a cloud computing configuration.

Non-transitory memory 406 may store a camera module 408, a scan control module 410, a coil connection module 412, and an IRD GUI 414. Camera module 408 may be configured to receive a respective video feed from the one or more cameras 424 and process each video feed. The processing may include analyzing each video feed to determine the current position of the table, subject, RF coil units, and/or the like. The processing may further include providing a live video feed to be included in one or more GUIs output for display on the at least one display device 420, such as on IRD GUI 414, as will be explained in more detail below.

Scan control module 410 may be configured to send commands to the MRI apparatus (e.g., to controller unit 25) in order to control aspects of a scan carried out by the MRI apparatus. Scan control module 410 may control aspects of the scan based on user input, which may be received via the IRD GUI 414, in some examples. For example, the IRD GUI 414 may include an initiate scanning button via which a user (e.g., the operator 210) may instruct the MRI apparatus to initiate scanning of a subject. The user input that is used to control aspects of the scan may be received via a GUI displayed on an operator console (e.g., operator console 304) that may include a scan prescription display panel via which a user may set parameters for the scan (e.g., whether parameter mapping is to be performed, if the scan is to include parallel imaging, etc.).

Coil connection module 412 may be configured to determine the status of each RF coil port of the table of the MRI apparatus (such as port 27). The status of each port may be selected from a list of possible statuses, including idle (e.g., no RF coil connected to the port), coil connected properly (e.g., an RF coil is connected to the port, and the RF coil is compatible with the port), and coil connected improperly (e.g., an RF coil is connected to the port, but the RF coil is not compatible with the port). The status of each port may be displayed via the IRD GUI 414. Coil connection module 412 may determine the status of each port based on the number of pins of the connector of the RF coil that are connected to the port. Based on the number of pins, the number of channels in the RF coil may be determined. Thus, the port may send signals to the coil connection module 412 indicating the number of connected pins and the coil connection module 412 may determine the number of channels in the RF coil based on the number of connected pins. Based on the number of channels in the RF coil, the coil connection module 412 may determine if the RF coil is compatible with the port. However, in some examples, additional information may be communicated from the RF coil to the coil connection module 412 via the port. For example, once a connection between the connector of the RF coil and the port has been established, the port may send signals to the coil connection module 412 indicating the model/type of RF coil (e.g., head and neck array, knee, etc.), manufacturer of the RF coil, and so forth. The RF coil may send this information directly via the connection between the connector and port (e.g., via the pins) or indirectly, such as via a radio frequency identification tag. The coil connection module 412 may store a look-up table in memory that indexes RF coil identity (identified by name, type, channel number, and/or manufacturer) to compatible ports for the specific MRI apparatus/table. Some RF coils may be compatible with only port, while other RF coils may be compatible with more than one port. Further, some RF coils, such as 48-channel RF coils, may demand connection with two ports simultaneously, which may also be noted in the look-up table.

In some embodiments, non-transitory memory 406 may include components disposed at two or more devices, which may be remotely located and/or configured for coordinated processing. In some embodiments, one or more aspects of non-transitory memory 406 may include remotely-accessible networked storage devices configured in a cloud computing configuration.

User input device 422 may comprise one or more of a touchscreen, a keyboard, a mouse, a trackpad, a motion sensing camera, or other device configured to enable a user to interact with and manipulate data within scan control device 402. In one example, user input device 422 may enable a user to make a selection of a scan protocol, adjust scan prescription settings, and the like, as well as initiate, pause, and adjust scanning.

Display device 420 may include one or more display devices utilizing virtually any type of technology. Display device 420 may be combined with processor 404, non-transitory memory 406, and/or user input device 422 in a shared enclosure, or may be peripheral display devices and may comprise a monitor, touchscreen, projector, or other display device known in the art, which may enable a user to view MRI images produced by the MRI apparatus, and/or interact with various data stored in non-transitory memory 406. For example, display device 420 may include IRD 302 and may display IRD GUI 414.

It should be understood that scan control device 402 shown in FIG. 2 is for illustration, not for limitation. Another appropriate image processing system may include more, fewer, or different components.

FIG. 5 shows a first example view 500 of an IRD GUI 501. IRD GUI 501 is an example of IRD GUI 414 and may be displayed on IRD 302. IRD GUI 501 may include three main display areas, including a first display area 502, a second display area 504, and third display area 506. The first display area 502 includes a live video feed 507 obtained from a camera positioned in the scan room (e.g., one of the one or more cameras 424). The live video feed 507 may depict an imaging subject, herein a patient 508, positioned on a table 510 of the MRI apparatus. The table 510 may be positioned in the home position, which may include the table 510 being coupled to, but fully external to, a bore 512 of the MRI apparatus. In the illustrated example, an RF coil 509 has been positioned on the patient 508, and thus is depicted in the live video feed 507.

The first display area 502 further includes an overlay to schematically illustrate components and information of interest not visible in the live video feed 507. The overlay may include a schematic depiction of RF coils embedded in the table 510, including a first posterior RF coil 514 and a second posterior RF coil 516. The overlay may additionally include, at least in some examples, landmark indicators. The landmark indicators may include a movable element, herein a handle 518, centered over a patient/anatomical landmark and a set of cross-hairs 519 (e.g., a vertical line and a horizontal line) extending out from the handle 518. The handle 518 may be repositioned in response to user input (e.g., the user may perform a drag-and-drop operation on the handle 518) and the set of cross-hairs 519 may move with the handle 518. The position of the handle 518 relative to the patient 508 may define the landmark, which may be used by the scan control device to set aspects of the scan (e.g., how far to move the table 510 into the bore 512 so that the landmark is centered in the bore 512).

The overlay displayed in the first display area 502 may further include a plurality of port status indicators. As shown in FIG. 5, the plurality of port status indicators includes a first port status indicator 520, a second port status indicator 522, a third port status indicator 524, and a fourth port status indicator 526. Each port status indicator may include an icon that has a visual appearance to indicate a current status of a respective RF coil port of the table, as determined by the coil connection module 412, for example. Each port status indicator may be positioned on the overlay to correspond to approximately where the port represented by that port status indicator is located on the table. For example, in the example shown in FIG. 5, the first port status indicator 520 includes an icon (herein a colored square) that indicates a status of a first port (e.g., labeled P1 on the first port status indicator 520), and is located in an upper left corner of the overlay in order to indicate the location of the first port is an upper left corner of the table 510. The second port status indicator 522 includes a similar icon indicating a status of a second port (e.g., labeled P2 on the second port status indicator 522) and is located in an upper right corner of the overlay, corresponding to the location of the second port positioned on an upper right corner of the table 510. The third port status indicator 524 also includes a similar icon indicating a status of a third port (e.g., labeled P3 on the third port status indicator 524) and is located in a lower right corner of the overlay, corresponding to the location of a third port positioned on a lower right corner of the table 510. The fourth port status indicator 526 includes the similar icon indicating a status of a fourth port (e.g., labeled P4 on the fourth port status indicator 526) and is located in a lower left corner of the overlay, corresponding to the location of the fourth port positioned on a lower left corner of the table 510.

In the first view 500, each port status indicator has a first visual appearance to indicate that each port currently has an idle status, e.g., is not connected to an RF coil. In the example shown, the first visual appearance is the square of the icon having a first color (e.g., gray), though other visual appearances are possible without departing from the scope of this disclosure.

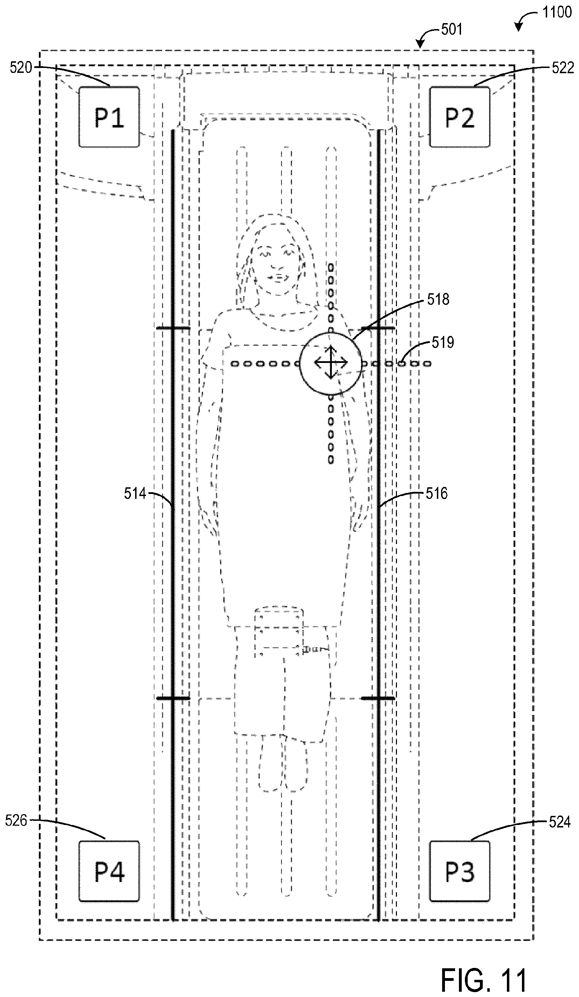

FIG. 11 shows another view 1100 of a portion of the IRD GUI 501. Specifically, FIG. 11 shows the first display area 502 with elements of the overlay shown in solid lines (e.g., the plurality of port status indicators, the depiction of the posterior RF coil elements, and the landmark indicators) and elements of the live camera feed shown in dashed lines. It is to be appreciated that the live camera feed may be replaced with a schematic depiction of the table and optionally an imaging subject without departing from the scope of this disclosure.

The second display area 504 may include various display elements to visualize information about the patient 508, the scan to be performed, and the RF coil ports. For example, the second display area 504 may include a first tile 530 that displays patient information (e.g., name, medical record number, date of birth), a second tile 532 that displays selected scan protocol(s) to be carried out when scanning the patient, and a menu 534 showing a selected patient position on the table. The menu 534 may be selectable so that the user may view other options for patient positioning and select a desired position.

The second display area 504 further includes a coil connection card 536 that displays compatibility information for each RF coil port. For example, the coil connection card 536 includes a first port compatibility region 538 that displays compatibility information for the first port. The coil connection card 536 further includes a second port compatibility region 540 that displays compatibility information for the second port, a third port compatibility region 542 that displays compatibility information for the second port, and a fourth port compatibility region 544 that displays compatibility information for the fourth port. The compatibility information that is displayed in each region may change based on the status of the corresponding port. In the first view 500, because each port is idle, the compatibility information may include information about each port's configuration, such as the maximum number of channels the port is configured to connect to and whether the port can accept receive-only RF coils or transmit/receive RF coils. For example, the text of the first port compatibility region 538 may include the port number (P1) to identify the port and "32ch RX" to indicate the first port is compatible with receive-only coils of 32 channels (or fewer). Further, in the first view 500, the coil connection card 536 has a first visual appearance to convey the idle status of each port (e.g., no highlighting, perimeter color, status indicators, etc.).

The second display area 504 may include additional display elements that convey selected patient parameters and table position information. For example, a plurality of display elements may display patient electrocardiogram (ECG) information (such as first ECG display element 546 and second ECG display element 548), patient pulse rate, patient respiration rate, etc. Further, a table position element 550 may display the current position of the patient table relative to a home position. Once a landmark is confirmed, the values displayed in the table position element 550 may change to the distance from the iso center where scanning will occur.

The third display area 506 may include a plurality of selectable user interface elements 552, such as buttons, that, when selected, cause various actions to occur. For example, the plurality of selectable user interface elements 552 may include a camera element that causes display or termination of the live video feed 507, a landmarking button that causes display of the landmark indicators, an ECG button that causes display of ECG waveforms of the patient (e.g., in the ECG display elements), etc. The plurality of selectable user interface elements 552 additionally includes a scan initiation element 554 that, when selected, causes the table 510 to move into the bore 512 so that scanning of the patient can commence.

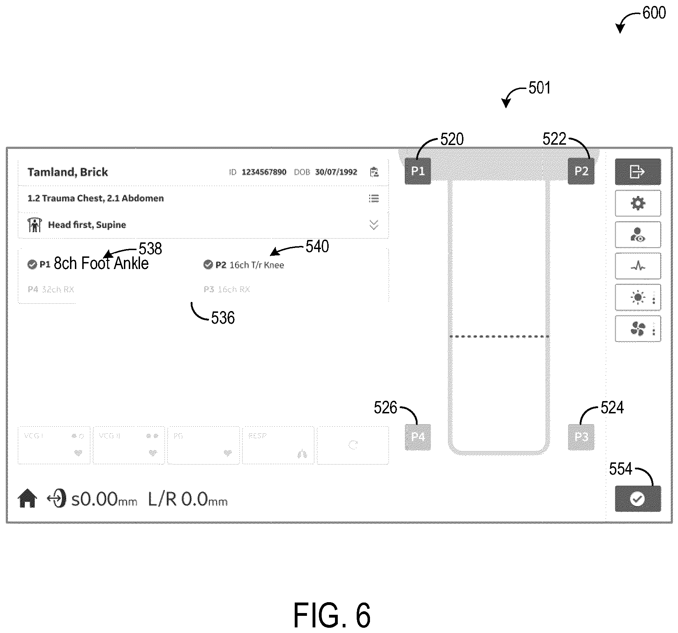

In some examples, the IRD GUI 501 may not include the live video feed 507. For example, the user may disable the live camera feed or the MRI apparatus may not include a camera configured to provide a live feed for inclusion in the IRD GUI 501. In such examples, the first display area 502 may include a schematic depiction of the table 510 that is the approximately same size and shape as the table 510, with the port status indicators included at the same positions shown in FIG. 5 (e.g., at the corners of the schematically-depicted table), as shown in FIGS. 6-8.

FIG. 6 shows the IRD GUI 501 in a second view 600. The second view 600 is identical to the first view 500, other than a schematic view of the table is shown instead of the live camera feed and the status of two of the ports has changed and thus the visual appearance of two of the port status indicators has changed in the second view 600 relative to the first view 500 and the information displayed in two of the port compatibility information regions has changed in the second view 600 relative to the first view 500. Specifically, a first RF coil has been connected to the first port and a second RF coil has been connected to the second port. The first RF coil may be compatible with the first port and the second RF coil may be compatible with the second port. The first port status indicator 520 thereby has a second visual appearance that indicates a proper/compatible connection between the first RF coil and the first port and the second port status indicator 522 has the second visual appearance that indicates a proper/compatible connection between the second RF coil and the second port. The second visual appearance may be different than the first visual appearance of the port status indicators of the first view 500, such as a different color (e.g., green instead of gray). Because the third and fourth ports remain idle, the third port status indicator 524 and the fourth port status indicator 526 still have the first visual appearance.

The coil connection card 536 is also updated to reflect the change in port status for the first and second ports. The first port compatibility region 538 includes a compatibility indicator (e.g., a green check mark) to indicate that the first RF coil is compatible with the first port. The first port compatibility region 538 further includes information on the first RF coil, e.g., the type of coil and number of channels of the first RF coil (e.g., 8-channel foot/ankle array). The second port compatibility region 540 likewise includes the compatibility indicator and information on the second RF coil (e.g., that the second RF coil is a 16-channel transmit/receive knee array).

In some examples, the coil connection card 536 in the second view 600 may be updated to have a second visual appearance relative to the coil connection card 536 in the first view 500. For example, the perimeter of the coil connection card 536 may be displayed in green in the second view 600 to indicate that each of the current RF coil-port connections is compatible. Further, as appreciated by FIG. 6, the text displayed in port compatibility regions of the coil connection card 536 corresponding to connected ports may have a different visual appearance than the text displayed in port compatibility regions of the coil connection card 536 corresponding to ports having an idle connection. For example, the text may be displayed in a different color, in bold, and/or in a larger font for the ports having a connection to an RF coil relative to ports having an idle connection.

Thus, the IRD GUI 501 may be updated when one or more ports are connected to a respective RF coil in order to change the visual appearance of the corresponding port compatibility indicator icon(s) and the coil connection card. In the second view 600, all detected RF coil-port connections are determined to be compatible. Thus, the scan initiation element 554 is depicted as being selectable (e.g., is displayed in color, with highlighting, or otherwise emphasized), as incompatibility issues that may hamper a successful diagnostic scan are not detected. It is to be appreciated that while FIG. 6 shows a schematic view of the table, the live video feed could be displayed in the second view 600, as shown in FIG. 5.

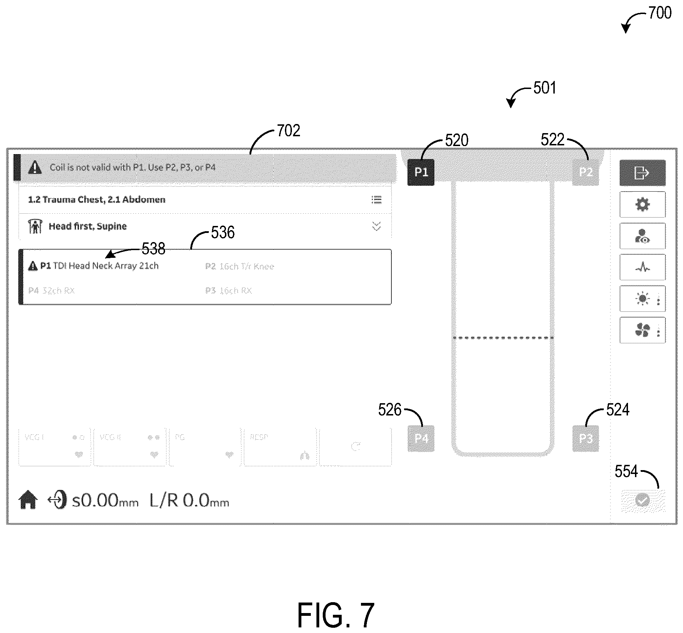

FIG. 7 shows the IRD GUI 501 in a third view 700. The third view 700 is identical to the first view 500, other than the schematic view of the table is shown instead of the live camera feed and the status of one of the ports has changed and thus the visual appearance of one of the port status indicators has changed in the third view 700 relative to the first view 500 and the information displayed in one of the port compatibility information regions has changed in the third view 700 relative to the first view 500. Specifically, a third RF coil has been connected to the first port. The third RF coil may be incompatible with the first port. The first port status indicator 520 thereby has a third visual appearance that indicates an improper connection between the third RF coil and the first port. The third visual appearance may be a specific color (e.g., red) that is different than the first visual appearance of the port status indicators and that is different than the second visual appearance of the port status indicators shown in the second view 600. Because the second, third, and fourth ports remain idle, the second port status indicator 522, the third port status indicator 524, and the fourth port status indicator 526 still have the first visual appearance.

The coil connection card 536 is also updated to reflect the change in port status for the first port. The first port compatibility region 538 includes an incompatibility indicator (e.g., a red triangle including an explanation point) to indicate that the third RF coil is incompatible with the first port. The first port compatibility region 538 further includes information on the third RF coil, e.g., the type of coil and number of channels of the third RF coil (e.g., 21-channel head/neck array).

In some examples, the coil connection card 536 in the third view 700 may be updated to have a third visual appearance relative to the coil connection card 536 in the first view 500. For example, the perimeter of the coil connection card 536 may be displayed in red in the third view 700 to indicate that at least one of the current RF coil-port connections is incompatible. Further, as appreciated by FIG. 7, the text displayed in port compatibility regions of the coil connection card 536 corresponding to connected ports may have a different visual appearance than the text displayed in port compatibility regions of the coil connection card 536 corresponding to ports having an idle connection. For example, the text may be displayed in a different color, in bold, and/or in a larger font for the ports having a connection to an RF coil relative to ports having an idle connection.

Additionally, the IRD GUI 501 in the third view 700 includes an alert icon 702 that provides further information about the incompatible coil-port connection. In the illustrated example, the alert icon 702 is in the form of a banner that extends across the top of the second display area 504 (e.g., obscuring the first tile 530). The alert icon 702 may have a visual appearance that contrasts with the visual appearance of the underlying elements of the IRD GUI, such as specific color or colors. The alert icon 702 may include text that conveys the incompatibility issue (e.g., the coil is not valid with the first port) and, when possible, a suggestion for one or more ports that could be used to connect to the RF coil.

Thus, the IRD GUI 501 may be updated when one or more ports are connected to a respective RF coil in order to change the visual appearance of the corresponding port compatibility indicator icon(s) and the coil connection card. In the third view 700, all detected RF coil-port connections are determined to be incompatible. Thus, the scan initiation element 554 is depicted as being unselectable (e.g., is not displayed in color, does not include highlighting, or otherwise is de-emphasized), as incompatibility issues that may hamper a successful diagnostic scan have been detected. As such, the user may not be able to initiate scanning of the patient until the coil connections have been adjusted to be compatible. It is to be appreciated that while FIG. 7 shows a schematic view of the table, the live video feed could be displayed in the third view 700, as shown in FIG. 5.

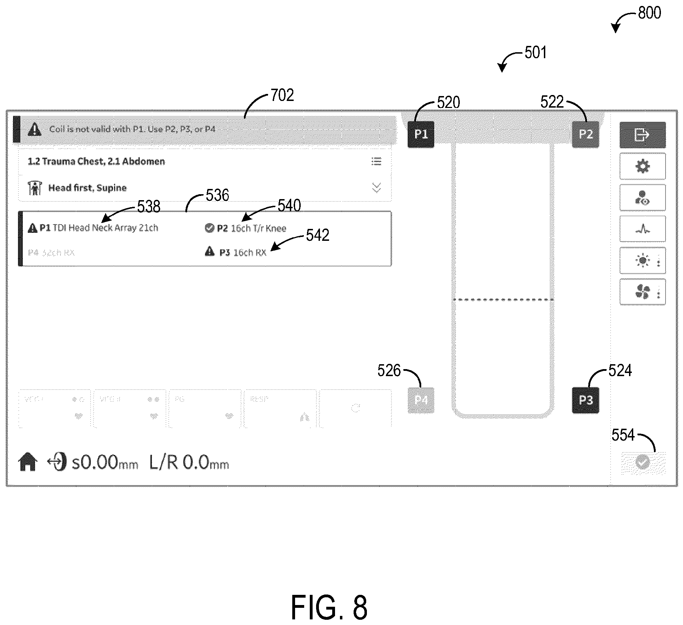

FIG. 8 shows the IRD GUI 501 in a fourth view 800. The fourth view 800 is identical to the first view 500, other than the schematic view of the table is shown instead of the live camera feed and the status of three of the ports has changed and thus the visual appearance of three of the port status indicators has changed in the fourth view 800 relative to the first view 500 and the information displayed in three of the port compatibility information regions has changed in the fourth view 800 relative to the first view 500. Specifically, a fourth RF coil has been connected to the first port, a fifth RF coil has been connected to the second port, and a sixth RF coil has been connected to the third port. The fourth RF coil may be incompatible with the first port, the fifth RF coil may be compatible with the second port, and the sixth RF coil may be incompatible with the third port. The first port status indicator 520 thereby has the third visual appearance (similar to the third visual appearance in the third view 700) that indicates an improper connection between the fourth RF coil and the first port. The second port status indicator 522 has the second visual appearance (similar to the second visual appearance in the second view 600) that indicates a proper connection between the fifth RF coil and the second port. The third port status indicator 524 has the third visual appearance that indicates an improper connection between the sixth RF coil and the third port. Because the fourth port remains idle, the fourth port status indicator 526 still has the first visual appearance.

The coil connection card 536 is also updated to reflect the change in port status for the first, second, and third ports. The first port compatibility region 538 includes the incompatibility indicator to indicate that the fourth RF coil is incompatible with the first port. The first port compatibility region 538 further includes information on the fourth RF coil, e.g., the type of coil and number of channels of the third RF coil (e.g., 21-channel head/neck array). The third port compatibility region 542 likewise includes the incompatibility indicator and information on the sixth RF coil. The second port compatibility region 540 includes the compatibility indicator to indicate the fifth RF coil is compatible with the second port and information on the fifth RF coil.

In some examples, the coil connection card 536 in the fourth view 800 may be updated to have the third visual appearance relative to the coil connection card 536 in the first view 500. For example, the perimeter of the coil connection card 536 may be displayed in red in the fourth view 800 to indicate that at least one of the current RF coil-port connections is incompatible. Further, as appreciated by FIG. 8, the text displayed in port compatibility regions of the coil connection card 536 corresponding to connected ports may have a different visual appearance than the text displayed in port compatibility regions of the coil connection card 536 corresponding to ports having an idle connection. For example, the text may be displayed in a different color, in bold, and/or in a larger font for the ports having a connection to an RF coil relative to ports having an idle connection.

Additionally, the IRD GUI 501 in the fourth view 800 includes the alert icon 702 that provides further information about one of the incompatible coil-port connections. In the illustrated example, the alert icon 702 includes text that conveys the incompatibility issue (e.g., the coil is not valid with the first port) and, when possible, a suggestion for one or more ports that could be used to connect to the RF coil. Because more than one incompatible coil-port connection has been detected, the alert icon 702 may include additional text that conveys the compatibility issue for the third port (not shown in FIG. 8), or the alert icon 702 may toggle between showing incompatibility information for each port with an incompatible connection.

Thus, the IRD GUI 501 may be updated when one or more ports are connected to a respective RF coil in order to change the visual appearance of the corresponding port compatibility indicator icon(s) and the coil connection card. In the fourth view 800, a portion of the detected RF coil-port connections are determined to be incompatible. Thus, the scan initiation element 554 is depicted as being unselectable (e.g., is not displayed in color, does not include highlighting, or otherwise is de-emphasized), as incompatibility issues that may hamper a successful diagnostic scan have been detected. As such, the user may not be able to initiate scanning of the patient until the coil connections have been adjusted to be compatible. It is to be appreciated that while FIG. 8 shows a schematic view of the table, the live video feed could be displayed in the fourth view 800, as shown in FIG. 5.

The third view 700 and the fourth view 800 of the IRD GUI 501 illustrated herein depict one type of coil connectivity incompatibility, namely the wrong type of RF coil for a given port. However, other incompatibility issues may be detected and conveyed via the IRD GUI 501, such as more than RF coil connected to a single coil, only one connector of a multi-connector RF coil being connected to a port, and a non-scanning coil being connected. Further, the text displayed in the alert icon 702 is non-limiting and other text may be presented. For example, rather than include text that states "Coil is not valid with P1," the text in the alert icon 702 may state "Coil is valid, but not on this port, Port: Port 1 (P1)" when the wrong type of RF coil is connected. Other text that may be included in an alert icon when an incompatible connection is detected include "Please reconnect the coil;" "Connected coil is not used for scanning. May require additional components;" "Wrong coil connected to port P3. Connect it to P2 or P4 instead;" "Multiple Coils are connected on a single port - [Coil name 1; Coil name 2];" "Only one connector (P1) of a two-connector coil is connected. Please connect with another port."

FIG. 9 illustrates a method 900 for scanning a patient with an MRI apparatus, such as the MRI apparatus 10 of FIG. 1. Method 900 is described with regard to the systems and components of FIGS. 1-4, though it should be appreciated that the method 900 may be implemented with other systems and components without departing from the scope of the present disclosure. Method 900 may be carried out according to instructions stored in non-transitory memory of a computing device, such as memory 406 of FIG. 4, and executed by a processor of the computing device, such as processor 404 of FIG. 2.

At 902, method 900 includes receiving an indication that an imaging subject, herein a patient, is positioned on a table of the MRI apparatus. For example, an operator of the MRI apparatus (e.g., a technologist, radiologist, etc.) may enter user input identifying the patient or otherwise indicating a scan of the patient is about to commence. As another example, the operator may move the table to a home position in order to position the patient on the table, and the table being in the home position may provide an indication that the patient is on the table (or is about to be positioned on the table).

At 904, an **IRD** GUI is output for display on a display device, such as an in-room display device positioned on a bore of the MRI apparatus (such as IRD 302 positioned on bore 202). The **IRD** GUI may present information relevant to patient positioning and scan set-up, including patient information, scan protocol information, and so forth. The IRD GUI may include a live camera feed showing the patient positioned on the table, with an overlay that includes landmark indicators and/or a plurality of port status indicators. When the patient is initially positioned on the table and before any RF coils have been connected to the MRI apparatus, each port status indicator of the IRD GUI may have a first visual appearance to convey that each port of the MRI apparatus is idle. For example, the IRD GUI that is displayed initially may be the IRD GUI the first view shown in FIG. 5. It is to be appreciated that the camera feed is optional and that when the camera feed is not included in the IRD GUI, the overlay including the port status indicators may still be displayed in the IRD GUI.

At 906, method 900 includes monitoring the coil-port connections and updating the IRD GUI when indicated, which is explained in more detail below with respect to FIG. 10. Briefly, each port of the MRI apparatus may be monitored so that when a connection between a port and an RF coil is established, the IRD GUI may be updated to reflect the connection and indicate if the connection is compatible or not compatible, so that the operator may correctly connect each desired RF coil prior to initiating the scan and leaving the scan room.

At 908, method 900 includes placing and/or adjusting a landmark and updating the IRD GUI accordingly. As explained above with respect to FIG. 5, the IRD GUI may include landmark indicators that designate a specific patient anatomical region as a landmark, to assist with patient positioning in the bore of the MRI apparatus and aspects of the scan. The landmark indicators may be placed and/or moved based on user input. In some examples, the landmark indicators (e.g., a set of cross-hairs and a handle) may be placed at a position on the IRD GUI corresponding to a location where the operator taps on the area of the IRD GUI that includes the live camera feed and overlay, and the operator may then drag the handle to a desired position. As another example, the landmark indicators may be placed on the IRD GUI based on the selected scan protocol and/or based on user input to the table (e.g., the operator may touch the table to set a vertical location of the landmark indicators), and then the operator may drag the handle to a desired position. It is to be appreciated that the landmark indicators may be positioned before or after the RF coils are plugged into the corresponding ports, or the landmark indicators may not be shown on the IRD GUI.

At 910, the scan is initiated when requested. For example, the IRD GUI may include a user interface element (e.g., the scan initiation element 554) that, when selected, causes the table to move into the bore of the MRI apparatus so that scanning can commence. Scanning of the patient may include playing out RF excitation pulses (e.g., with the RF body coil unit 15 and/or one or more of the surface/local RF coils) and magnetic field gradient pulses (e.g., with the gradient coil unit 13) with specific timings and in a specific sequence to prepare contrast and encode spatial information into MR signals that are detected by the one or more surface/local RF coils (such as RF coil unit 14). The detected MR signals are then transformed into images. In some examples, scanning may commence with localizer scans to ensure the patient is positioned properly before full diagnostic scanning begins. Method 900 then ends.

It is to be appreciated that during display of the IRD GUI explained above with respect to FIG. 9, an operator console, such as operator console 304, may display a scan control GUI that is different than the IRD GUI. The scan control GUI may present information and accept user input related to the scan, in order to allow selection of a scan protocol, view localizer scan images, set scan parameters, view scan progress, view diagnostic images, and the like. In some examples, coil and port information may be displayed via a coil connection tab of the scan control GUI. However, the coil connection tab of the scan control GUI may include only coil names and port numbers (e.g., 21ch head neck array connected to port 1) and may not include compatibility information or visual representations of where the ports are located. For example, the scan control GUI may not include an image of the table or patient.

FIG. 10 illustrates a method 1000 for monitoring RF coil-port connections in an MRI apparatus, such as the MRI apparatus 10 of FIG. 1. Method 1000 is described with regard to the systems and components of FIGS. 1-4, though it should be appreciated that the method 1000 may be implemented with other systems and components without departing from the scope of the present disclosure. Method 1000 may be carried out according to instructions stored in non-transitory memory of a computing device, such as memory 406 of FIG. 4, and executed by a processor of the computing device, such as processor 404 of FIG. 2. In some examples, method 1000 may be carried out as part of method 900, such as at 906 of method 900.

At 1002, method 1000 includes outputting an IRD GUI with indications of available RF coil ports. The IRD GUI may be the IRD GUI 501 explained above with respect to FIGS. 5-8. The indications of the available RF coil ports may include port status indicators displayed on a live camera feed overlay, as indicated at 1004. However, in some examples, the port status indicators may be displayed on a schematic depiction of the table of the MRI apparatus, as shown in FIGS. 6-8. The port status indicators may be displayed at locations that approximately correspond to the actual locations of the RF coil ports on the table of the MRI apparatus. When no RF coils are connected, each port status indicator may have a first visual appearance to indicate that each port is currently idle, as shown in FIG. 5.

The indications of the available RF coil ports may further include a coil connection card, as indicated at 1006. The coil connection card may display information about each RF coil port, such as the port number and the type(s) of RF coils the port is configured to accept. When no RF coils are connected, the coil card may have a first visual appearance to indicate that each port is currently idle, as shown in FIG. 5.

At 1008, method 1000 detects if an RF coil has been connected to a port. An RF coil connected to a port may be detected based on signals output by the port, such as signals indicating the number of pins of the connector of the RF coil that are connected to the port. In some examples, additional signals may be output by the port or otherwise received at the computing device that indicate the make, model, etc., of the RF coil.

If no connection between an RF coil and a port is detected, method 1000 loops back to 1002 to continue to output the IRD GUI showing the available (e.g., idle) ports. If a coil-port connection is detected, method 1000 proceeds to 1012 to determine if the coil and the port are compatible based on the number of pins of the connector of the RF coil that are connected to the port and/or based on the make, model, etc., of the RF coil. The number of connected pins may be used to determine the number of channels of the RF coil. The computing device may determine which port(s) of the MRI apparatus are compatible with the RF coil based on the number of channels and in some examples further based on the make, model, etc., of the RF coil, using a look-up table stored in memory, for example (as explained above with respect to FIG. 4).

If the RF coil and the port are determined to be compatible, method 1000 proceeds to 1014 to update the IRD GUI to indicate a compatible coil-port connection. Updating the IRD GUI to indicate the compatible coil-port connection may include updating the port status indicator that represents the port and updating the coil connection card, as indicated at 1016. The port status indicator that represents the port of the detected coil-port connection may be adjusted to have a second visual appearance that is different than the first visual appearance, such as the visual appearance of the first port status indicator 520 of FIG. 6. Similarly, the coil connection card may be updated to include a compatibility indicator in the region of the connected port and display information on the connected RF coil, as shown in FIG. 6. In some examples, updating the IRD GUI to indicate the compatible coil-port connection may include updating or maintaining the scan initiation element, as indicated at 1018, so that the scan initiation element is shown as being selectable.

At 1020, method 1000 includes determining if a request to initiate scanning has been received (e.g., via user input to the scan initiation element). If a request to initiate scanning has been received, method 1000 ends. If a request to initiate scanning has not been received, method 1000 continues to 1002 to output the IRD GUI with the indications of available ports (and, in this instance, also including the updated port status indicator and coil connection card).

Returning to 1012, if it is determined that the RF coil and the port that the RF coil is connected to are not compatible (e.g., if the RF coil-port connection is an incompatible connection), method 1000 proceeds to 1022 to update the IRD GUI to indicate an incompatible coil-port connection. Updating the IRD GUI to indicate the incompatible coil-port connection may include updating the port status indicator that represents the port and updating the coil connection card, as indicated at 1024. The port status indicator that represents the port of the detected coil-port connection may be adjusted to have a third visual appearance that is different than the first visual appearance and the second visual appearance of the port status indicator, such as the visual appearance of the first port status indicator 520 of FIG. 7. Similarly, the coil connection card may be updated to have an updated visual appearance (e.g., a change in the color of the perimeter of the card) that is different than the visual appearance of the coil connection card when the ports are idle or only compatible connections are detected. The coil connection card may also be updated to include an incompatibility indicator in the region of the connected port and to display information on the connected RF coil, as shown in FIG. 7. Additionally, the IRD GUI may be updated to include an alert icon, such as alert icon 702, that conveys additional information about the incompatible connection. In some examples, updating the IRD GUI to indicate the incompatible coil-port connection may include updating or maintaining the scan initiation element, as indicated at 1026, so that the scan initiation element is shown as not being selectable. Method 1000 then proceeds to 1008 to continue to monitor for new or updated RF coil-port connections.

Thus, disclosed herein is a specialized system (e.g., the scan control device in combination with the MRI apparatus) for real-time coil guidance in MRI environments. The system integrates hardware and software components to provide dynamic, visual feedback on coil connectivity status via the disclosed IRD GUI, improving the efficiency and accuracy of MRI setup procedures. The system directly interfaces with the RF coil ports of the MRI apparatus, actively monitoring the physical connection status of each coil in real-time. This involves continuous electrical signal processing to detect coil presence and compatibility.

For MRI systems equipped with in-room cameras, the IRD GUI overlays digital information onto the live camera feed. This demands precise spatial calibration and real-time image processing to accurately map coil port locations onto the video stream.

The IRD GUI disclosed herein is an adaptive user interface, and the system dynamically generates and updates the GUI based on the current state of coil connections. This involves real-time data processing to interpret coil connection states, dynamic generation of visual elements (colors, shapes, icons) corresponding to connection status, and immediate update of the display/GUI in response to physical changes in coil connections.

Further, the system performs real-time analysis of connected coils against the specifications of each port, determining if a coil is correctly connected or mismatched. This involves maintaining a database of coil and port specifications, executing compatibility algorithms to cross-reference connected coils with port requirements, and generating appropriate visual and textual feedback based on the analysis results.

The system disclosed herein employs a combination of visual cues (colors, shapes, icons) and textual information to convey complex status information in an easily interpretable format. This demands design and implementation of a coherent visual language for status representation, development of algorithms to translate technical status data into appropriate visual and textual cues, and implementation of accessibility features to ensure the information is perceivable by users with various visual capabilities.

Further still, the system actively identifies and alerts users to incorrect coil connections or potential issues, involving continuous monitoring and analysis of coil connection data, implementation of error detection algorithms, and generation and display of context-appropriate alert messages. It is to be appreciated that a person may not be able to mentally identify whether a coil-port connection is a compatible connection or an incompatible connection, as RF coil connectors and ports may not be labeled and different RF coils may have connectors that are visually similar to each other and that can be connected to a variety of different ports, even if the RF coil is not actually compatible with that port.

Additionally, the system is configured to function on both camera-equipped and non-camera MRI systems, demanding development of adaptive rendering algorithms to suit different display contexts and implementation of a flexible software architecture that can accommodate varying hardware configurations.

A technical effect of determining a connection status for each of a plurality of ports, each port of the plurality of ports configured to couple an RF coil to an MRI apparatus and generating a GUI that includes a port status indicator for each port, where each port status indicator has a visual appearance that is based on that port's connection status, is that a user may be informed of incorrect or correct RF coil-port connections so that the user can rectify any incorrect connections before scanning starts. In doing so, scan retakes may be reduced and image quality may be increased, which may increase the efficiency of the computing device(s) controlling the MRI apparatus and generating images.

The disclosure also provides support for a system, comprising: a display device, one or more processors, and memory storing instructions executable by the one or more processors to: determine a connection status for each of a plurality of ports, each port of the plurality of ports configured to couple a radiofrequency (RF) coil to a magnetic resonance imaging (MRI) apparatus, generate a graphical user interface (GUI) that includes a port status indicator for each port, each port status indicator having a visual appearance that is based on that port's connection status, and output the GUI for display on the display device. In a first example of the system, the display device is an in-room display device positioned on a bore of the MRI apparatus. In a second example of the system, optionally including the first example, determining the connection status for each port includes determining that each port has an idle connection status, and wherein generating the GUI includes generating the GUI with a coil connection card that displays compatibility information about each port of the plurality of ports and setting each port status indicator to have a first visual appearance that indicates the idle connection status. In a third example of the system, optionally including one or both of the first and second examples, determining the connection status for each port includes determining that a first connection status for a first port of the plurality of ports has changed from the idle connection status to a compatible connection status, and updating the GUI to change the visual appearance of a first port status indicator from the first visual appearance to a second visual appearance to indicate the compatible connection status, the compatible connection status reflecting a first RF coil connected to the first port is compatible with the first port. In a fourth example of the system, optionally including one or more or each of the first through third examples, the system further comprises: updating the coil connection card to include a compatibility indicator for the first port and display configuration information about the first RF coil. In a fifth example of the system, optionally including one or more or each of the first through fourth examples, determining the connection status for each port includes determining that a first connection status for a first port of the plurality of ports has changed from the idle connection status to an incompatible connection status, and updating the GUI to change the visual appearance of a first port status indicator from the first visual appearance to a third visual appearance to indicate the incompatible connection status, the incompatible connection status reflecting a first RF coil connected to the first port is incompatible with the first port. In a sixth example of the system, optionally including one or more or each of the first through fifth examples, the system further comprises: updating the coil connection card to include an incompatibility indicator for the first port and display configuration information about the first RF coil. In a seventh example of the system, optionally including one or more or each of the first through sixth examples, the system further comprises: updating the GUI to include an alert icon that displays incompatibility information about the first RF coil. In an eighth example of the system, optionally including one or more or each of the first through seventh examples, the plurality of ports is positioned on a table of the MRI apparatus, wherein the GUI includes a first display area configured to display a live video feed of the table, and wherein each port status indicator is displayed as an overlay on the live video feed, each port status indicator positioned at a location on the overlay that corresponds to a location of a corresponding port on the table.

The disclosure also provides support for a method, comprising: determining a connection status for each of a plurality of ports of a magnetic resonance imaging (MRI) apparatus, each port of the plurality of ports configured to couple a radiofrequency (RF) coil to the MRI apparatus, generating a graphical user interface (GUI) that includes a port status indicator for each port, each port status indicator having a visual appearance that is based on that port's connection status, and outputting the GUI for display on a display device. In a first example of the method, the display device is positioned on a bore of the MRI apparatus. In a second example of the method, optionally including the first example, determining the connection status for each of the plurality of ports of the MRI apparatus comprises determining that a first port of the plurality of ports has an idle connection status and that a second port of the plurality of ports has an incompatible connection status, the idle connection status indicating no RF coil is coupled to the first port, the incompatible connection status indicating that a first RF coil coupled to the second port is not compatible with the second port, and wherein generating the GUI includes generating the GUI with a first port status indicator corresponding to the first port having a first visual appearance and a second port status indicator corresponding to the second port having a second visual appearance, different than the first visual appearance. In a third example of the method, optionally including one or both of the first and second examples, generating the GUI includes generating the GUI with a coil connection card that displays compatibility information about the first port and configuration information about the first RF coil. In a fourth example of the method, optionally including one or more or each of the first through third examples, generating the GUI includes generating the GUI with an alert icon that displays incompatibility information about the first RF coil.

The disclosure also provides support for a method, comprising: determining, based on signals output by a first port of a plurality of ports positioned on a table of a magnetic resonance imaging (MRI) apparatus, that a first RF coil is connected to the first port, determining, based on the signals, that the first RF coil is not compatible with the first port, updating, in response to determining that the first RF coil is not compatible with the first port, a graphical user interface (GUI) to include a first port status indicator for the first port having a second visual appearance that is different than a first visual appearance of the first port status indicator, the first port status indicator displayed on the GUI with the first visual appearance when the first port is determined to be idle, and outputting the GUI for display on a display device positioned on a bore of the MRI apparatus. In a first example of the method, the GUI includes one or more additional port status indicators each corresponding to a respective remaining port of the plurality of ports and each having a visual appearance based on whether an RF coil is connected to the corresponding respective remaining port. In a second example of the method, optionally including the first example, the plurality of ports is positioned on the table of the MRI apparatus, wherein the GUI includes a first display area configured to display a live video feed of the table, and wherein each port status indicator is displayed as an overlay on the live video feed, each port status indicator positioned at a location on the overlay that corresponds to a location of a corresponding port on the table. In a third example of the method, optionally including one or both of the first and second examples, updating, in response to determining that the first RF coil is not compatible with the first port, the GUI includes updating a visual appearance of a coil connection card of the GUI and updating port compatibility information displayed within the coil connection card to indicate that the first RF coil is not compatible with the first port. In a fourth example of the method, optionally including one or more or each of the first through third examples, updating, in response to determining that the first RF coil is not compatible with the first port, the GUI to include an alert icon that suggests a different port of the plurality of ports that is compatible with the first RF coil. In a fifth example of the method, optionally including one or more or each of the first through fourth examples, the first visual appearance includes the first port status indicator having a first color and the second visual appearance includes the first port status indicator having a second color.

As used herein, an element or step recited in the singular and preceded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

The following claims particularly point out certain combinations and sub-combinations regarded as novel and non-obvious. These claims may refer to "an" element or "a first" element or the equivalent thereof. Such claims should be understood to include incorporation of one or more such elements, neither requiring nor excluding two or more such elements. Other combinations and sub-combinations of the disclosed features, functions, elements, and/or properties may be claimed through amendment of the present claims or through presentation of new claims in this or a related application. Such claims, whether broader, narrower, equal, or different in scope to the original claims, also are regarded as included within the subject matter of the present disclosure.

## Claims

1. A system, comprising:
a display device (420);
one or more processors (404); and
memory (406) storing instructions executable by the one or more processors (404) to:
determine (906) a connection status for each of a plurality of ports (27), each port of the plurality of ports (27) configured to couple a radiofrequency (RF) coil (14) to a magnetic resonance imaging (MRI) apparatus (10);
generate (1002) a graphical user interface (GUI) (501) that includes a port status indicator (520, 522, 524, 526) for each port, each port status indicator (520, 522, 524, 526) having a visual appearance that is based on that port's connection status; and
output (906) the GUI (501) for display on the display device (420).

2. The system of claim 1, wherein the display device is an in-room display device (302) positioned on a bore (202) of the MRI apparatus (10).

3. The system of claim 1, wherein determining the connection status for each port includes determining that each port has an idle connection status, and wherein generating the GUI includes generating (1006) the GUI with a coil connection card (536) that displays compatibility information about each port of the plurality of ports and setting (1004) each port status indicator to have a first visual appearance that indicates the idle connection status.

4. The system of claim 3, wherein determining the connection status for each port includes determining that a first connection status for a first port of the plurality of ports has changed from the idle connection status to a compatible connection status, and updating (1014, 1016) the GUI to change the visual appearance of a first port status indicator from the first visual appearance to a second visual appearance to indicate the compatible connection status, the compatible connection status reflecting a first RF coil connected to the first port is compatible with the first port.

5. The system of claim 4, further comprising updating (1016) the coil connection card to include a compatibility indicator for the first port and display configuration information about the first RF coil.

6. The system of claim 3, wherein determining the connection status for each port includes determining that a first connection status for a first port of the plurality of ports has changed from the idle connection status to an incompatible connection status, and updating (1022, 1024) the GUI to change the visual appearance of a first port status indicator from the first visual appearance to a third visual appearance to indicate the incompatible connection status, the incompatible connection status reflecting a first RF coil connected to the first port is incompatible with the first port.

7. The system of claim 6, further comprising updating (1024) the coil connection card to include an incompatibility indicator for the first port and display configuration information about the first RF coil.

8. The system of claim 6, further comprising updating the GUI to include an alert icon (702) that displays incompatibility information about the first RF coil.

9. The system of claim 1, wherein the plurality of ports is positioned on a table (26) of the MRI apparatus (10), wherein the GUI (501) includes a first display area configured to display a live video feed (507) of the table, and wherein each port status indicator is displayed as an overlay on the live video feed, each port status indicator positioned at a location on the overlay that corresponds to a location of a corresponding port on the table.

10. A method, comprising:
determining (906) a connection status for each of a plurality of ports (27) of a magnetic resonance imaging (MRI) apparatus (10), each port of the plurality of ports configured to couple a radiofrequency (RF) coil (14) to the MRI apparatus (10);
generating (1002, 1014, 1022) a graphical user interface (GUI) (501) that includes a port status indicator (520, 522, 524, 526) for each port, each port status indicator having a visual appearance that is based on that port's connection status; and
outputting (906) the GUI for display on a display device (420).

11. The method of claim 10, wherein the display device is positioned on a bore (202) of the MRI apparatus (10).

12. The method of claim 10, wherein determining the connection status for each of the plurality of ports of the MRI apparatus comprises determining that a first port of the plurality of ports has an idle connection status and that a second port of the plurality of ports has an incompatible connection status, the idle connection status indicating no RF coil is coupled to the first port, the incompatible connection status indicating that a first RF coil coupled to the second port is not compatible with the second port, and wherein generating the GUI includes generating the GUI with a first port status indicator corresponding to the first port having a first visual appearance and a second port status indicator corresponding to the second port having a second visual appearance, different than the first visual appearance.

13. The method of claim 12, wherein generating the GUI includes generating the GUI with a coil connection card (536) that displays compatibility information about the first port and configuration information about the first RF coil.

14. The method of claim 12, wherein generating the GUI includes generating the GUI with an alert icon (702) that displays incompatibility information about the first RF coil.
